# EUROPEAN PATENT SPECIFICATION

(11) **EP 4 526 893 B1**
(45) Date of publication and mention of the grant of the patent: **03.12.2025**
(21) Application number: 23725236.6
(22) Date of filing: 09.05.2023
(51) Int. Cl.: G16H 40/40, G16H 30/20, G16H 40/67, H04L 67/12, H04L 69/24

(54) **ACTIVE DEVICE FOR COMPATIBILITY CHECK OF SYSTEM COMPONENTS AND CONNECTED DEVICES**
AKTIVE VORRICHTUNG ZUR KOMPATIBILITÄTSPRÜFUNG VON SYSTEMKOMPONENTEN UND ANGESCHLOSSENEN VORRICHTUNGEN
DISPOSITIF ACTIF DE VÉRIFICATION DE COMPATIBILITÉ DE COMPOSANTS DE SYSTÈME ET DE DISPOSITIFS CONNECTÉS

(30) Priority: 19.05.2022 EP 22174366
(43) Date of publication of application: 26.03.2025
(73) Proprietor: Koninklijke Philips N.V., 5656 AG Eindhoven (NL)
(72) Inventor: VOGTMEIER, Gereon, 5656 AG Eindhoven (NL); CHAKRABARTI, Biswaroop, 5656 AG Eindhoven (NL); LEUSSLER, Christoph Günther, 5656 AG Eindhoven (NL)
(74) Representative: Philips Intellectual Property & Standards
(86) International application number: PCT/EP2023/062171
(87) International publication number: WO 2023/222437

(56) References cited:
- US-A1- 2016 371 442

## Description

### FIELD OF THE INVENTION

The present invention relates to medical imaging, and in particular to a compatibility testing apparatus and a system for compatibility check of a component connectable to a medical imaging system, to a medical imaging system, and to a method for operating the compatibility testing apparatus.

### BACKGROUND OF THE INVENTION

Today the imaging systems are connected to several additional components and/or systems that provide additional signals and/or information via analogue and/or digital interfaces. However, the compatibility of interfaces and the compatibility with the protocols is not always ensured. A second source of uncertainty and also a risk is the use of spare parts that are not always original supplier parts but sometimes OEM replacements where quality is not always guaranteed by the supplier. Even the availability of additional functionalities or less functionalities could lead to situations of limited compatibility and as a consequence a reduction of functionality, image quality, and/or system availability and uptime. US 2016/371442 A1 describes a compatibility checking mechanism can include a reader, a checking mechanism, a writer and an approver. The document does not disclose performance of compatibility tests.

### SUMMARY OF THE INVENTION

It is thus an object of the present invention to enable a stable and/or safe operation of a medical imaging system with a connected component.

The object of the present invention is solved by the subject-matter of the independent claims. Further embodiments and advantages of the invention are incorporated in the dependent claims. Furthermore, it shall be noted that all embodiments of the present invention concerning a method might be carried out with the order of the steps as described, nevertheless this has not to be the only and essential order of the steps of the method as presented herein. The method disclosed herein can be carried out with another order of the disclosed steps without departing from the respective method embodiment, unless explicitly mentioned to the contrary hereinafter.

According to a first aspect of the present invention, there is provided a compatibility testing apparatus for compatibility check of a component connectable to a medical imaging system. The compatibility testing apparatus comprises a connectivity module and a compatibility check engine. The connectivity module is connectable to a compatibility test interface of the medical imaging system. The compatibility check engine is configured to perform one or more compatibility tests to determine a compatibility between the connected component and the medical imaging system and to generate a result associated with the one or more compatibility tests.

The inventors of the present invention have found out that an issue for the supplier of the system is the responsibility to deliver an agreed quality of the product and/or service, which for the imaging systems like magnetic resonance imaging (MRI), computed tomography (CT), digital X-ray radiogrammetry (DXR), positron emission tomography (PET), Single-photon emission computed tomography (SPECT), image guided interventional treatment (IGIT), and ultrasound sound (US) could lead to lower image quality and even a false diagnose. For example, a non-standard message originating from one component may cause a side effect in another component and either have an unintended consequence in the resulting image generation or cause a malfunction in the second component. Imaging modalities that involve ionizing radiation may exceed safe limits of radiation exposure to the patient and/or the operator due to such an unintended interaction. The inventors of the present invention have also found out that the availability of additional functionalities or less functionalities may lead to situations of limited compatibility and as a consequence a reduction of functionality, image quality, and/or system availability and uptime. This may happen due to software upgrades in future with e.g., different versions.

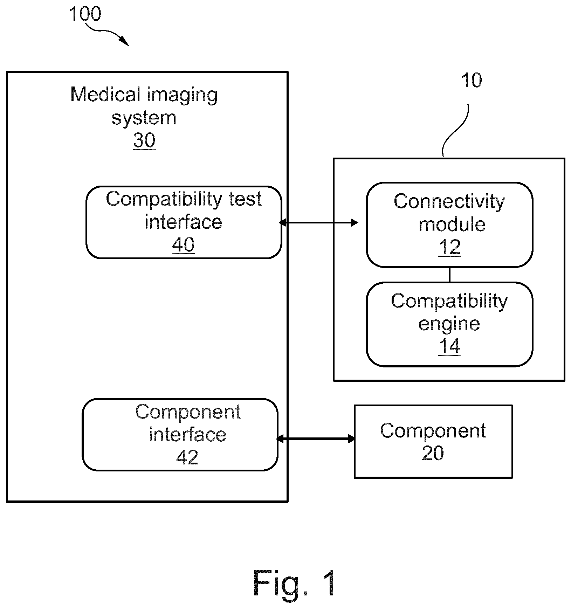

In order to address at least one of the above-described issues, a compatibility testing apparatus is provided in the present disclosure to test e.g., the compatibility and the availability of necessary functionality, which may be beneficial for the service engineer. The compatibility testing apparatus may be an external device, a permanent control unit in an imaging system, or part of the interface to the external device with additional "blocking function". This would guarantee also the identification and check of changes after connection of different external devices, replacement of components, and/or software upgrades that even could be enabled/disabled temporarily by pay per use models. The real-time check may enable also the certification of ordered functionality with a defined quality level in the operating environment. This will be explained in detail hereinafter and in particular with respect to the examples shown in FIGS. 1 to 3.

According to the first aspect of the present invention, the medical imaging system is configured to exchange data with the connected component via a data interface in accordance with a communication protocol. The one or more compatibility tests comprise a test of compatibility of the connected component with the data interface and/or the communication protocol.

In the prior art document US 2016/371442 A1, the compatibility check is defined by reading content from a memory of a device, and the acceptance or deny of the usage of components is based on a "compatibility data entry in the memory", which is, however, a static (pre-defined) information.

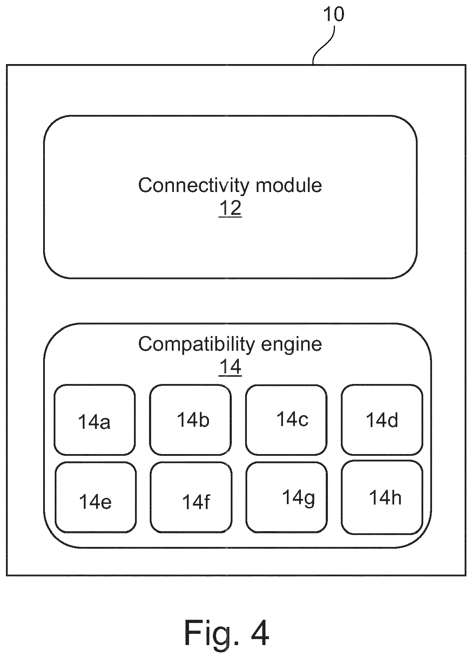

To that end, the compatibility testing apparatus is proposed to be capable of performing an active testing of interfaces and protocols. Accordingly, the compatibility testing apparatus can perform an active check on timing and data sequence during data exchange to identify dynamic problems. **In** this way, as will be described in detail hereinafter and in particular with respect to the example shown in FIG. 4, the compatibility testing apparatus as disclosed herein may actively measure or test e.g., based on action-reaction pattern in a real time scenario. The compatibility testing apparatus as disclosed herein may differentiate between individual functions that can be enabled or disabled. The compatibility testing apparatus as disclosed herein may even check not only base-functionality but also test performance and the achievable quality level.

Additionally, the imaging systems are usually connected to several additional components and/or systems that may provide additional signals and/or information via analogue and/or digital interfaces, which may have different voltage levels of the interface signals. This exemplary compatibility test may ensure the compatibility of interfaces and the compatibility with the protocols.

According to an embodiment of the present invention, the compatibility check engine is configure to perform one or more of the following:
- monitoring a data communication between the medical imaging system and the connected component, and to determining whether the data communication follows a predetermined communication protocol;
- communicating with the connected component with a message to elicit a response, and comparing the elicited response with a desired response to determine whether the connected component is compatible with the medical imaging system; and
- triggering an event representing a change or an activity that takes place in an operating environment of the medical imaging system, monitoring a reaction of the connected component to the event, and determining whether the connected component is compatible with the medical imaging system based on an action-reaction pattern.

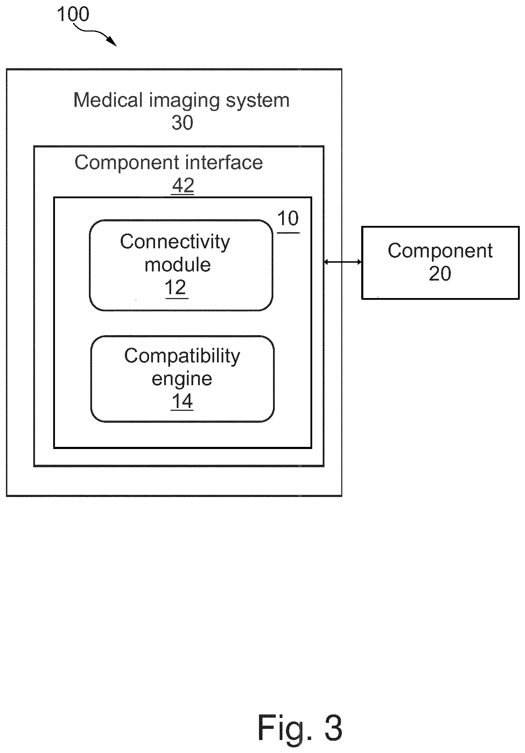

This will be explained in detail hereinafter and in particular with respect to the example shown in Fig. 3.

According to an embodiment of the present invention, the one or more compatibility tests comprise a functionality check to determine whether the connected component supports an additional functionality.

The availability of additional functionalities or less functionalities may lead to situations of limited compatibility and as a consequence a reduction of functionality. This exemplary compatibility check may enable the certification of ordered functionality with a defined quality level in the operating environment.

According to an embodiment of the present invention, in response to determining that the connected component does not supports the additional functionality, the compatibly testing apparatus is configured to perform one or more of:
- disabling the additional functionality for the connected component;
- blocking a communication between the connected component and the medical imaging system; and
- configuring the medical imaging system in a different setting to support the additional functionality for the connected component.

According to an embodiment of the present invention, in response to determining that the connected component supports the additional functionality, the compatibly testing apparatus is configured to perform one or more of:
- enabling the additional functionality for the connected component;
- performing a validated message translation to enable the additional functionality in the connected component, when it is determined that the connected component has a functionality similar to the additional functionality but with a different communication protocol; and
- providing information about the connected component and a setting of the medical imaging system.

According to an embodiment of the present invention, the one or more compatibility tests comprise a measurement of a performance characteristic of the medical imaging system with the connected component to determine whether the measured performance characteristic is within a defined quality level.

This will be explained in detail hereinafter and in particular with respect to the example shown in Fig. 3.

According to an embodiment of the present invention, the compatibility check engine is configured to perform one or more of the following:
- receiving sensor data obtained from an imaging phantom, determining a performance of the medical imaging system based on the sensor data, and determining whether the performance of the medical imaging system fulfils the defined quality level; and
- receiving image data of an imaging phantom acquired by the medical imaging system, determining an image quality of the acquired image data, and to determine whether the image quality fulfils the defined quality level.

According to an embodiment of the present invention, the one or more compatibility tests comprise a simulation of out-of-range data to check for reaction of the connected component.

Simulating out of range data to check for reaction of the connected component may allow for test of system security as the component may operate in a kind of "data firewall attack and detection mode".

According to an embodiment of the present invention, the one or more compatibility tests comprise a test of compatibility of a known and certified third-party component to generate a result of the compatibility test.
In other words, the compatibility with known and certified third-party systems and devices may be checked. The real-time tests may provide more reliable results than just a "device name" of the connected device.

According to an embodiment of the present invention, the compatibility check engine is configured to determine a risk level based on the result associated with the one or more compatibility tests.

According to an embodiment of the present invention, in response to determining that the determined risk level is within an allowable risk range, the component is allowable to operate with the medical imaging system. In response to determining that the determined risk level exceeds an allowable risk range, the compatibility check engine is configured to generate an error message and/or to trigger an action to reduce the risk level.

Depending on the risk level, additional functionality and features may also be allowed to operate.

For example, the compatibility check engine may block the communication between the medical imaging system and the connected component.

According to a second aspect of the present invention, there is provided a system for compatibility check of a component connected to a medical imaging system. The system comprises a compatibility testing apparatus according to the first aspect and any associated example and an imaging phantom with a sensor arrangement. The sensor arrangement comprises one or more sensors configured to acquire sensor data indicative of a performance of the medical imaging system.

In this way, the compatibility testing apparatus may be used as a service tool with some additional physical measurement functionalities.

According to a third aspect of the present invention, there is provided a medical imaging system, comprising a compatibility testing apparatus according to the first aspect or a system according to the second aspect.

According to a fourth aspect of the present invention, there is provided a method for operating a compatibility testing apparatus according to the first aspect for compatibility check of a component connected to a medical imaging system, the method comprising:
- connecting a connectivity module of the compatibility testing apparatus to a data interface of the medical imaging system; and
- performing one or more compatibility tests to determine whether the connected component is compatible with the medical imaging system.
As used herein, the term "modality" refers to a recording unit, for example an X-ray unit, a computer tomography scanner, etc. for recording data, such as examination image data. Such a unit may include a data processing unit for processing and local storage, for a limited time, of the data, that is to say of the examination image data.

As used herein, the terms "first", "second", "third", and the like are intended to distinguish between different objects, but do not indicate a particular order of the objects. For example, a first module, a second module, and the like are intended to distinguish between different modules, but do not indicate a particular order of the modules.

It should be appreciated that all combinations of the foregoing concepts and additional concepts discussed in greater detail below (provided such concepts are not mutually inconsistent) are contemplated as being part of the inventive subject matter disclosed herein.

These and other aspects of the invention will be apparent from and elucidated with reference to the embodiment(s) described hereinafter.

### BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the invention.
Fig. 1 illustrates an exemplary compatibility testing apparatus for compatibility check of a component connected to a medical imaging system.
Fig. 2 illustrates another exemplary compatibility testing apparatus for compatibility check of a component connected to a medical imaging system.
Fig. 3 illustrates a further exemplary compatibility testing apparatus for compatibility check of a component connected to a medical imaging system.
Fig. 4 illustrates another exemplary compatibility testing apparatus for compatibility check of a component connected to a medical imaging system.
Fig. 5 illustrates an exemplary system for compatibility check of a component connected to a medical imaging system.
Fig. 6 illustrates a flowchart describing a method for operating a compatibility testing apparatus.

### DETAILED DESCRIPTION OF EMBODIMENTS

Fig. 1 illustrates an exemplary compatibility testing apparatus 10 for compatibility check of a component 20 connected to a medical imaging system 30. The exemplary compatibility testing apparatus 10, the component 20, and the medical imaging system 30 form a system 100. In some examples, as shown in FIG. 1, the component 20 may be an external device (indicated with solid lines), such as MR coils, electrocardiography (ECG) devices, respiration sensors, microphone, electronics of the MR system, and the like, which may be connected to a magnetic resonance imaging (MRI) scanner. The component 20 may be connected to a data interface of the medical imaging system 30, such as component interface 42 shown in FIG. 1. In some examples, the component 20 may be a system component (not shown), i.e., a component within the medical imaging system 30. The component 20 may comprise a software module with a block of machine instructions and data stored in a memory (not shown). The software module may execute in a programmable processor coupled to the memory. The software module may interact with one or more other software modules to perform some desired function.

The medical imaging system 30 of the illustrated example may comprise any appropriate modality for recording data, such as imaging data. Examples of the medical imaging system 30 may include, but are not limited to, a CT scanner for computed tomography, an MRI scanner, a digital subtraction angiography appliance, an X-ray imaging appliance for digital radiography, a PET scanner for positron emission tomography, a SPECT scanner for photon emission computed tomography, and an ultrasound (US) imaging appliance.

The compatibility testing apparatus 10 of the illustrated example comprises a connectivity module 12 and a compatibility check engine 14. In general, the compatibility testing apparatus 10 may comprise various physical and/or logical components for communicating and manipulating information, which may be implemented as hardware components (e.g. computing devices, processors, logic devices), executable computer program instructions (e.g. firmware, software) to be executed by various hardware components, or any combination thereof, as desired for a given set of design parameters or performance constraints. Although FIG. 1 may show a limited number of components by way of example, it can be appreciated that a greater or a fewer number of components may be employed for a given implementation.

In some implementations, the compatibility testing apparatus 10 may be embodied as, or in, a device or apparatus, such as a server, workstation, or mobile device. The compatibility testing apparatus 10 may comprise one or more microprocessors or computer processors, which execute appropriate software. For example, the compatibility check engine 14 of the compatibility testing apparatus 10 may be embodied by one or more of these processors. The software may have been downloaded and/or stored in a corresponding memory, e.g. a volatile memory such as RAM or a nonvolatile memory such as flash. The software may comprise instructions configuring the one or more processors to perform the functions described herein.

It is noted that the compatibility testing apparatus 10 may be implemented with or without employing a processor, and also may be implemented as a combination of dedicated hardware to perform some functions and a processor (e.g. one or more programmed microprocessors and associated circuitry) to perform other functions. For example, the functional units of the compatibility testing apparatus 10, e.g. the interconnectivity module 12 and the compatibility check engine may be implemented in the device or apparatus in the form of programmable logic, e.g. as a Field-Programmable Gate Array (FPGA). In general, each functional unit of the compatibility testing apparatus may be implemented in the form of a circuit.

The connectivity module 12 of the illustrated example may allow operative connection of the exemplary compatibility testing apparatus 10 to the medical imaging system 30 for data exchange. The connectivity module 12 may comprise a data bus system or a communication network. Although FIG. 1 may show a limited number of connectivity module by way of example, it can be appreciated that a plurality of connectivity modules may be employed for a given implementation. For example, the plurality of connectivity modules may be connected to the different physical interfaces (e.g., data buses) and could be configured according to the system configuration.

The compatibility check engine 14 of the illustrated example may be configured to monitor data transmitted between the component 20 and the medical imaging system 30 and/or to measure a performance characteristic of the medical imaging system 30 with the connected component 20 for compatibility check.

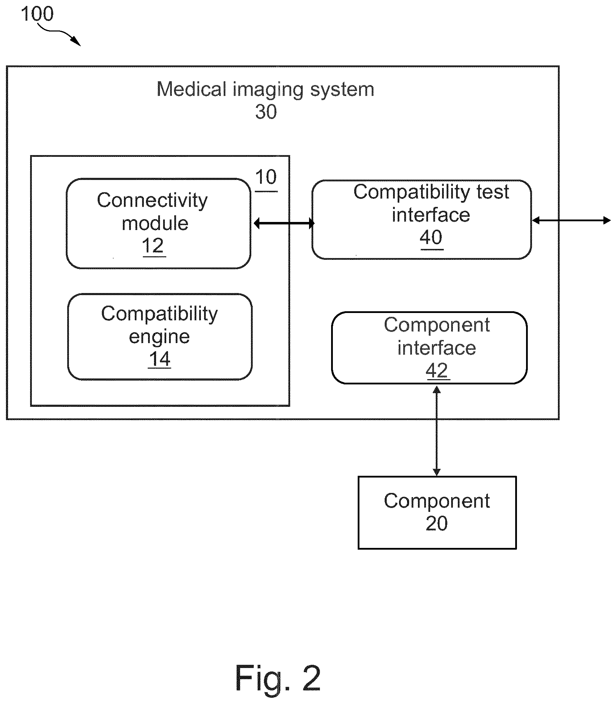

In the illustrated example shown in Fig.1, the exemplary compatibility testing apparatus 10 is an external device which is connectable to the medical imaging system 30 via e.g., a data bus system or a communication network. Alternatively, as shown in Fig. 2, the exemplary compatibility testing apparatus 10 may be a part of the medical imaging system 30. The exemplary compatibility testing apparatus 10 may report a result of one or more compatibility tests via a compatibility test interface 40, which may give a technician the opportunity to easily access the compatibility of the connected component. The compatibility test interface 40 may work on standardized interfaces. The exemplary compatibility testing apparatus 10 may provide a standardized series of diagnostic trouble codes via the compatibility test interface 40, which allow a person to rapidly identify and remedy the compatibility issues with the connected component. As a further example, as shown in FIG. 3, the exemplary compatibility testing apparatus 10 may be embodied in the interface, such as the component interface 42, between the medical imaging system 30 and the component 20, i.e., the external device, and may have the function to disconnect the external device.

In general, the compatibility check engine 14 may be configured to perform one or more compatibility tests to determine a compatibility between the connected component and the medical imaging system and to generate a result associated with the one or more compatibility tests.

The medical imaging system 30 is configured to exchange data with the connected component 20 via a data interface in accordance with a communication protocol. The one or more compatibility tests comprise a test of compatibility of the connected component with the data interface and/or the communication protocol. Accordingly, the compatibility testing apparatus is configured to perform an active testing of interfaces and protocols. The compatibility testing apparatus can perform an active check on timing and data sequence during data exchange to identify dynamic problems. For example, as will be described in detail hereinafter, the compatibility testing apparatus may actively measure or test e.g., based on action-reaction pattern in a real time scenario. The compatibility testing apparatus may differentiate between individual functions that can be enabled/disabled. The compatibility testing apparatus may even check not only base-functionality but also test performance and the achievable quality level. Compare to the prior art, in which the acceptance or deny of the usage of components is based on a static and pre-defined information, the compatibility testing apparatus as disclosed herein is configured to perform active compatibility testing and monitoring to avoid errors and detect dynamic problems.

In some examples, the one or more compatibility tests may comprise a functionality check to determine whether the connected component 20 supports an additional functionality. In response to determining that the connected component does not supports the additional functionality, the compatibly testing apparatus may be configured to disable the additional functionality for the connected component or configure the medical imaging system in a different setting to support the additional functionality for the connected component. In response to determining that the connected component supports the additional functionality, the compatibly testing apparatus may be configured to enable the additional functionality for the connected component. When it is determined that the connected component has a functionality similar to the additional functionality but with a different communication protocol, the compatibility check engine may be configured to perform a validated message translation to enable the additional functionality in the connected component. The compatibility check engine may be configured to provide information about the connected component and a setting of the medical imaging system. In this way, the component may be used for similar settings of the imaging system.

In some examples, the one or more compatibility tests may comprise a measurement of a performance characteristic of the medical imaging system 30 with the connected component 20 to determine whether the measured performance characteristic is within a defined quality level.

In some examples, the one or more compatibility tests may comprise a simulation of out-of-range data to check for reaction of the connected component.

In some examples, the one or more compatibility tests may comprise a test of compatibility of a known and certified third-party component to generate a result of the compatibility test.

In some examples, the compatibility check engine may be configured to determine a risk level based on the result associated with the one or more compatibility tests. The capability check engine 14 may provide information about the risks. Depending on the identified risk level the functionality and the added feature is allowed to operate and/or generate error/information messages may be generated.

The above-described exemplary compatibility tests may be executed with one or more different modules. For example, Fig. 4 illustrates a further example of the compatibility testing apparatus 10 for compatibility check of a component 20 connected to a medical imaging system 30. The compatibility check engine 14 of the illustrated example comprise a plurality of modules for performing compatibility check. Although Fig. 3 may show a limited number of modules by way of example, it can be appreciated that a greater or a fewer number of modules may be employed for a given implementation. The function of each module may be implemented in hardware, firmware, software, or a combination thereof. The compatibility check engine 14 may exchange data with the medical imaging system 30 over an interface protocol.

In some examples, as shown in Fig. 4, the compatibility check engine 14 may comprise a first module 14a configured to communicate with the connected component 20 with a message to elicit a response, and to compare the elicited response with a desired response to determine whether the connected component is capable of operating in the medical imaging system 30. For example, the first module 14a may be connected to the data interfaces to monitor the answers given by connected component 20 based on certain trigger conditions. The first module 14a may be implemented as an analyser to inspect or otherwise gather data regarding the exchange of data between the component 20 and the medical imaging system 30 via the interface protocol. The first module 14a may inspect the answers to reveal inefficiencies, errors, or other issues occurring between the component 20 and the medical imaging system 30 to e.g., allow for stability and reliability analysis. The first module 14a may actively communicate with the connected component 20 with messages to elicit desired responses.

In some examples, as shown in FIG. 4, the compatibility check engine 14 may comprise a second module 14b configured to trigger an event representing a change or an activity that takes place in an operating environment of the medical imaging system 30. The second module 14b is further configured to monitor a reaction of the connected component 20 to the event, and to determine whether the connected component is capable of operating in the medical imaging system based on an action-reaction pattern. For example, the event may include a software update on the medical imaging system. For example, the event may include a change in hardware of the medical imaging system. The second module 14b may check an information pattern (e.g., data pattern, parameter pattern, etc.) and/or a timing pattern of the reaction of the connected component to determine whether the connectable component is capable of operating in the medical imaging system.

In some examples, as shown in Fig. 4, the compatibility check engine 14 may comprise a third module 14c configured to perform a functionality check to determine whether the connected component 20 supports an additional functionality, and in response to the determination that the connected component 20 supports the additional functionality, to enable the additional functionality in the connected component 20 and/or in the medical imaging system 30. In order to enable the additional functionality in the medical imaging system 30, the medical imaging system 30 may be re-configured in a different way. Alternatively, a feedback signal may be provided to the medical imaging system 30. For example, the third module 14c may comprise a certified function enabler that allows additional functionality when compatibility and defined operating condition would be confirmed in the actual configuration. For example, enabled additional software feature requiring interaction with hardware might be installed (after upgrade) in only selected cases. In case where the connected devices are not directly compatible due to protocol differences, but otherwise have similar functionality, the third module 14c may allow validated message translation to enable additional functions in the connected devices, subject to validity of such an operation agreed upon by the manufacturers.

In some examples, as shown in Fig. 4, the compatibility check engine 14 may comprise a fourth module 14d configured to monitor a data communication between the medical imaging system and the connected component, and to determine whether the data communication follows a predetermined communication protocol. For example, the fourth module 14d may detect one or more of abnormal commands, data ranges, non-defined formats, etc. In case of abnormal commands, data ranges, and or nonstandard data formats originating from a device, such data would be logged and may be sent to the intended device while the latter is in a safe/service mode to quantify the possible effect of such a communication. Typical device interactions recorded by passive listening would be used to recreate the condition for a typical scan/imaging operation and the radiation exposure and image quality would be analysed. When all parameters are acceptable, the combination of the devices and the software settings will be certified for safe operation. *Depending on the findings different actions could be triggered. Either a downgrade configuration to ensure a safe operation or a "certified operation" in the actual setting. Range limits could define the quality level and with that also the certified performance in case all tests are performed within the defined quality specification.*

*In some examples, as shown in* *Fig. 4**,* the compatibility check engine 14 may comprise a fifth module 14e configured to receive a component identifier from the connected component. The fifth module 14e is further configured to determine whether the connected component is a known and certified third-party component, and to perform the compatibility check to obtain a test result. In other words, the fifth module 14e may check the compatibility of the medical imaging system with known and certified third-party systems and devices with real-time tests to have a more reliable results than just the component identifier of the connected component. In some examples, connected device and/or software upgrade as a payed feature might be allowed to work only if compatibility is given. For example, it is possible to provide a function that the system can enable/disable the additional feature based on compatibility check and payment/order information to have a business control linked to the check (which has to be done before enabling).

In some examples, as shown in Fig. 4, the compatibility check engine 14 may comprise a sixth module 14f configured to receive sensor data obtained from an imaging phantom. The sixth module 14f is configured to determine a performance of the medical imaging system based on the sensor data, and to determine whether the performance of the medical imaging system fulfils a quality criterion and/or to provide a feedback about a performance parameter of the medical imaging system 30 with the connected component 20. For example, the sixth module 14f may allow for testing radiation exposure to parts of the phantom itself. The measured data, such as radiation exposure, may be compared with an allowable range of these measured parameters to determine whether the performance of the medical imaging system fulfils the quality criterion. Alternatively or additionally, the sixth module 14f may provide a feedback about a performance parameter of the medical imaging system 30 with the connected component 20.

In some examples, as shown in Fig.4, the compatibility check engine 14 comprises a seventh module 14g configured to receive image data of an imaging phantom acquired by the medical imaging system. The seventh module 14g is configured to determine an image quality of the acquired image data, and to determine whether the image quality fulfils a defined quality level. For example, the seventh module 14g may be configured to for testing an image quality, such as contrast, resolution, and noise levels in the resulting image to determine whether the image quality fulfils a defined quality level.

In some examples, as shown in Fig.4, the compatibility check engine 14 may comprise an eight module 14h configured to receive sensor data acquired by a sensor monitoring an operating environment of the medical imaging system, and to determine whether the connected component is capable of operating in the medical imaging system with a defined quality level in the monitored operating environment. The device may be tested under different influencing parameters. Examples of the influencing environmental condition may include, but are not limited to, heat, humidity and/or bad power condition or cooling condition.

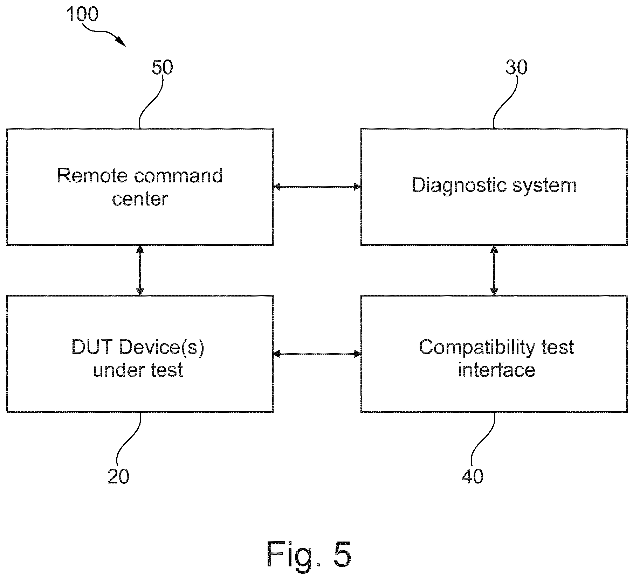

Fig. 5 illustrates an exemplary system for compatibility check of a component connected to a medical imaging system. The exemplary system 100 comprises a component 20, also referred to as device under test (DUT) in FIG. 5, a diagnostic system 30 with a compatibility test interface 40, and a remote command centre 50.

The diagnostic system 30 may be an MRI scanner. MRI is a non-invasive procedure that is essential for disease detection, diagnosis, and treatment management. The MRI system will be discussed in detail hereinafter by way of example. However, it will be appreciated the following description is also applicable to other modalities, such as CT, PET, SPECT, IGIT, DXR, and US.

The DUT device 20 may be any external device, such as MR coils, electrocardiography (ECG) devices, respiration sensors, microphone, electronics of the MR system, etc., which may be connected to a magnetic resonance imaging (MRI) scanner. In some cases, smart connected devices connected to the patient bed or MR scanner are FDA certified as a standalone equipment but may show up incompatibility in combination with other devices. RF coils from different vendors may come up to be applied on different systems by using smart interfaces or by wireless function. MR system installations are not identical with respect to inner RF screen wave propagation und may show up signal integrity issues.

The compatibility testing may be guided by an operator from a remote operation centre 50. The compatibility testing apparatus 10 as described hereinbefore may be embodied in the diagnostic system 30. The MR system may be equipped with a compatibility test interface 40. The DUT device may be fixed and located at defined positions, which are controlled by the system. An interactive test software may be provided to guide the user through the test procedure. The local operator may connect the sensing elements to the DUT device. For example, a RF coil may be tested using a special phantom and holder. Artefacts may be detected and identified for B0 homogeneity, B1 transmit field compatibility, signal integrity, and the like. The compatibility testing apparatus may discriminate the MRI imaging artefact level with respect to compatibility guidelines including local sensors for compatibility. For certain tests, an automated test procedure may be activated. The local operator may follow a bot which is controlled by a local state machine and cloud service data. When the test is finished and passed, the device under test or combination of different devices and their compatibility may receive a dedicated certificate for compatibility. The DUT device may then be used for certain settings of the imaging system. The certificate may be stored in the cloud and allow to make use of the device for a defined period of time. The remote or automated testing may be supported by three-dimensional sensing (3D) for positioning and monitoring.

The compatibility testing may be performed following a test routine selected from a predefined list of test modes, such as "minimum required", "normal operation", and "test for options/upgrades". Depending on the selected test mode, one or more modules 14a-14h shown in FIG. 3 may be activated to perform one or more compatibility checks. Each compatibility test may include a protocol standard, the completeness of the commands implementation, and/or the data ranges and allowed/not-allowed values. For example, in order to test system security, the connected device may operate in a kind of "data firewall attack and detection mode" when the second module 14b simulates out of range data to check for reaction of other connected devices. For this test, the safety of the imaging system/patient has to be ensured. Therefore, no active scan operation should be carried out when such safety test is started.

In idle mode of the system operation of the diagnostic system 30, one or more different test modes may be started. For example, in the idle mode of the system operation, the test mode "minimum required" may be frequently run to ensure tracking of stable operation. The test mode "test for options/upgrades" may only be executed after any change in hardware software and/or at a regular predefined schedule.

In the scan mode of the diagnostic system 30, the compatibility testing apparatus 10 may be configured to be in a listening mode to monitor the data exchange between the DUT device 20 and the diagnostic system 30 to detect abnormal commands, data ranges, non-defined formats, etc. If e.g., abnormal commands, data ranges, and/or nonstandard data formats originating from a device are detected, such data may be logged and may be sent to the intended device while the latter is in a safe/service mode to quantify the possible effect of such a communication. Typical device interactions recorded by passive listening may also be used to recreate the condition for a typical scan/imaging operation. The radiation exposure and image quality may be analysed. When all parameters are acceptable, the combination of the devices and the software settings will be certified for safe operation

Depending on the findings different actions could be triggered. In some examples, a downgrade configuration may be performed to ensure safe operation or a "certified operation" in actual settings. Range limits may be used define the quality level and with that also the certified performance in case all tests are performed within the defined quality specification. In case of component replacement that have impact on the performance the identified problem could lead to a simple warning, to a functional downgrade, or to an uncertified operation.

All findings might have impact on service and maintenance but also on the business-model as payments are connected to agreed contracts and operation conditions. For example, a replacement with a non-certified component might have impact on the guaranteed image quality and the service promise that a vendor gives to the customer. So such a device could be used as a neutral instance to report on the actual status of the system and the linked quality certification and if within/out agreement with the signed contract conditions.

This could include service by third parties and components by OEMs that might have a dedicated certification, such as trusted by the original supplier but with different specification level. Also different function levels might be certified by different component suppliers - and could be identified and checked by this system.

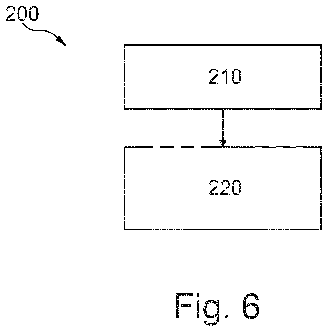

Fig. 6 illustrates a flowchart describing a method 200 for operating a compatibility testing apparatus as described herein for compatibility check of a component connected to a medical imaging system. At block 210, the connectivity module of the compatibility testing apparatus is connected to a compatibility test interface of the medical imaging system. At block 220, the connected compatibility test interface performs one or more compatibility tests as described herein to determine whether the connected component is compatible with the medical imaging system.

All definitions, as defined and used herein, should be understood to control over dictionary definitions, definitions in documents, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

**In** another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

## Claims

1. A compatibility testing apparatus (10) for compatibility check of a component (20) connectable to a medical imaging system (30), the compatibility testing apparatus comprising:
- a connectivity module (12); and
- a compatibility check engine (14);
wherein the connectivity module is connectable to the medical imaging system;
wherein the compatibility check engine is configured to perform one or more compatibility tests to determine a compatibility between the connected component and the medical imaging system and to generate a result associated with the one or more compatibility tests;
wherein the medical imaging system is configured to exchange data with the connected component via a data interface (42) in accordance with a communication protocol; and
wherein the one or more compatibility tests comprise a test of compatibility of the connected component with the data interface and/or the communication protocol.

2. The compatibility testing apparatus according to claim 1,
wherein the compatibility check engine is configured to perform one or more of the following:
- monitoring a data communication between the medical imaging system and the connected component, and to determining whether the data communication follows a predetermined communication protocol;
- communicating with the connected component with a message to elicit a response, and comparing the elicited response with a desired response to determine whether the connected component is compatible with the medical imaging system; and
- triggering an event representing a change or an activity that takes place in an operating environment of the medical imaging system, monitoring a reaction of the connected component to the event, and determining whether the connected component is compatible with the medical imaging system based on an action-reaction pattern.

3. The compatibility testing apparatus according to any one of the preceding claims,
wherein the one or more compatibility tests comprise a functionality check to determine whether the connected component supports an additional functionality.

4. The compatibility testing apparatus according to claim 3,
wherein in response to determining that the connected component does not supports the additional functionality, the compatibly testing apparatus is configured to perform one or more of:
- disabling the additional functionality for the connected component;
- blocking a communication between the connected component and the medical imaging system; and
- configuring the medical imaging system in a different setting to support the additional functionality for the connected component.

5. The compatibility testing apparatus according to claim 3 or 4,
wherein in response to determining that the connected component supports the additional functionality, the compatibly testing apparatus is configured to perform one or more of:
- enabling the additional functionality for the connected component;
- performing a validated message translation to enable the additional functionality in the connected component, when it is determined that the connected component has a functionality similar to the additional functionality but with a different communication protocol; and
- providing information about the connected component and a setting of the medical imaging system.

6. The compatibility testing apparatus according to any one of the preceding claims,
wherein the one or more compatibility tests comprise a measurement of a performance characteristic of the medical imaging system with the connected component to determine whether the measured performance characteristic is within a defined quality level.

7. The compatibility testing apparatus according to claim 6,
wherein the compatibility check engine is configured to perform one or more of the following:
- receiving sensor data obtained from an imaging phantom, determining a performance of the medical imaging system based on the sensor data, and determining whether the performance of the medical imaging system fulfils the defined quality level; and
- receiving image data of an imaging phantom acquired by the medical imaging system, determining an image quality of the acquired image data, and to determine whether the image quality fulfils the defined quality level.

8. The compatibility testing apparatus according to any one of the preceding claims,
wherein the one or more compatibility tests comprise a simulation of out-of-range data to check for reaction of the connected component.

9. The compatibility testing apparatus according to any one of the preceding claims,
wherein the one or more compatibility tests comprise a test of compatibility of a known and certified third-party component to generate a result of the compatibility test.

10. The compatibility testing apparatus according to any one of the preceding claims,
wherein the compatibility check engine is configured to determine a risk level based on the result associated with the one or more compatibility tests.

11. The compatibility testing apparatus according to claim 10,
wherein in response to determining that the determined risk level is within an allowable risk range, the additional functionality is allowable to operate with the medical imaging system; and
wherein in response to determining that the determined risk level exceeds an allowable risk range, the compatibility check engine is configured to generate an error message and/or to trigger an action to reduce the risk level.

12. A system (100) for compatibility check of a component connected to a medical imaging system, comprising:
- a compatibility testing apparatus according to any one of the preceding claims; and
- an imaging phantom with a sensor arrangement;
wherein the sensor arrangement comprises one or more sensors configured to acquire sensor data indicative of a performance of the medical imaging system.

13. A medical imaging system (30), comprising:
- a compatibility testing apparatus according to any one of claims 1 to **11** or a system according to claim 12.

14. A method (200) for operating a compatibility testing apparatus according to claim 1 for compatibility check of a component connected to a medical imaging system, the method comprising:
- connecting a connectivity module of the compatibility testing apparatus to a compatibility test interface of the medical imaging system; and
- performing one or more compatibility tests to determine whether the connected component is compatible with the medical imaging system.

## Patentansprüche

1. Kompatibilitätstesteinrichtung (10) zur Kompatibilitätsprüfung einer Komponente (20), die an ein medizinisches Bildgebungssystem (30) anschließbar ist, wobei die Kompatibilitätstesteinrichtung Folgendes umfasst:
- ein Konnektivitätsmodul (12); und
- eine Kompatibilitätsprüf-Engine (14);
wobei das Konnektivitätsmodul an das medizinische Bildgebungssystem anschließbar ist;
wobei die Kompatibilitätsprüf-Engine dazu konfiguriert ist, einen oder mehrere Kompatibilitätstests durchzuführen, um eine Kompatibilität zwischen der angeschlossenen Komponente und dem medizinischen Bildgebungssystem zu bestimmen, und ein dem einen oder den mehreren Kompatibilitätstests zugeordnetes Ergebnis zu erzeugen;
wobei das medizinische Bildgebungssystem dazu konfiguriert ist, Daten mit der angeschlossenen Komponente über eine Datenschnittstelle (42) gemäß einem Kommunikationsprotokoll auszutauschen; und
wobei der eine oder die mehreren Kompatibilitätstests einen Kompatibilitätstest der angeschlossenen Komponente mit der Datenschnittstelle und/oder dem Kommunikationsprotokoll umfassen.

2. Kompatibilitätstesteinrichtung nach Anspruch 1,
wobei die Kompatibilitätsprüf-Engine dazu konfiguriert ist, eines oder mehrere der Folgenden durchzuführen:
- Überwachen einer Datenkommunikation zwischen dem medizinischen Bildgebungssystem und der angeschlossenen Komponente und Bestimmen, ob die Datenkommunikation einem vorbestimmten Kommunikationsprotokoll folgt;
- Kommunizieren mit der angeschlossenen Komponente mit einer Nachricht, um eine Antwort hervorzurufen, und Vergleichen der hervorgerufenen Antwort mit einer gewünschten Antwort, um zu bestimmen, ob die angeschlossene Komponente mit dem medizinischen Bildgebungssystem kompatibel ist; und
- Auslösen eines Ereignisses, das eine Änderung oder eine Aktivität darstellt, die in einer Betriebsumgebung des medizinischen Bildgebungssystems stattfindet, Überwachen einer Reaktion der angeschlossenen Komponente auf das Ereignis und, basierend auf einem Aktions-Reaktions-Muster, Bestimmen, ob die angeschlossene Komponente mit dem medizinischen Bildgebungssystem kompatibel ist.

3. Kompatibilitätstesteinrichtung nach einem der vorstehenden Ansprüche,
wobei der eine oder die mehreren Kompatibilitätstests eine Funktionalitätsprüfung umfassen, um zu bestimmen, ob die angeschlossene Komponente eine zusätzliche Funktionalität unterstützt.

4. Kompatibilitätstesteinrichtung nach Anspruch 3,
wobei als Reaktion auf das Bestimmen, dass die angeschlossene Komponente die zusätzliche Funktionalität nicht unterstützt, die Kompatibilitätstesteinrichtung dazu konfiguriert ist, eines oder mehrere der Folgenden durchzuführen:
- Deaktivieren der zusätzlichen Funktionalität für die angeschlossene Komponente;
- Blockieren einer Kommunikation zwischen der angeschlossenen Komponente und dem medizinischen Bildgebungssystem; und
- Konfigurieren des medizinischen Bildgebungssystems in einer unterschiedlichen Einstellung, um die zusätzliche Funktionalität für die angeschlossene Komponente zu unterstützen.

5. Kompatibilitätstesteinrichtung nach Anspruch 3 oder 4,
wobei als Reaktion auf Bestimmen, dass die angeschlossene Komponente die zusätzliche Funktionalität unterstützt, die Kompatibilitätstesteinrichtung dazu konfiguriert ist, eines oder mehrere der Folgenden durchzuführen:
- Aktivieren der zusätzlichen Funktionalität für die angeschlossene Komponente;
- Durchführen einer validierten Nachrichtenübersetzung, um die zusätzliche Funktionalität in der angeschlossenen Komponente zu aktivieren, wenn bestimmt wird, dass die angeschlossene Komponente eine Funktionalität ähnlich der zusätzlichen Funktionalität aufweist, jedoch mit einem unterschiedlichen Kommunikationsprotokoll; und
- Bereitstellen von Informationen über die angeschlossene Komponente und eine Einstellung des medizinischen Bildgebungssystems.

6. Kompatibilitätstesteinrichtung nach einem der vorstehenden Ansprüche,
wobei der eine oder die mehreren Kompatibilitätstests eine Messung eines Leistungsmerkmals des medizinischen Bildgebungssystems mit der angeschlossenen Komponente umfassen, um zu bestimmen, ob das gemessene Leistungsmerkmal innerhalb eines definierten Qualitätsniveaus liegt.

7. Kompatibilitätstesteinrichtung nach Anspruch 6,
wobei die Kompatibilitätsprüf-Engine dazu konfiguriert ist, eines oder mehrere der Folgenden durchzuführen:
- Empfangen von Sensordaten, die von einem Bildgebungs-Phantom erhalten wurden, Bestimmen einer Leistung des medizinischen Bildgebungssystems basierend auf den Sensordaten und Bestimmen, ob die Leistung des medizinischen Bildgebungssystems das definierte Qualitätsniveau erfüllt; und
- Empfangen von Bilddaten eines Bildgebungs-Phantoms, die von dem medizinischen Bildgebungssystem erhoben werden, Bestimmen einer Bildqualität der erhobenen Bilddaten und Bestimmen, ob die Bildqualität das definierte Qualitätsniveau erfüllt.

8. Kompatibilitätstesteinrichtung nach einem der vorstehenden Ansprüche,
wobei der eine oder die mehreren Kompatibilitätstests eine Simulation von Daten außerhalb des Bereichs umfassen, um eine Reaktion der angeschlossenen Komponente zu prüfen.

9. Kompatibilitätstesteinrichtung nach einem der vorstehenden Ansprüche,
wobei der eine oder die mehreren Kompatibilitätstests einen Kompatibilitätstest einer bekannten und zertifizierten Drittanbieterkomponente umfassen, um ein Ergebnis des Kompatibilitätstests zu erzeugen.

10. Kompatibilitätstesteinrichtung nach einem der vorstehenden Ansprüche,
wobei die Kompatibilitätsprüf-Engine dazu konfiguriert ist, basierend auf dem Ergebnis, das dem einen oder den mehreren Kompatibilitätstests zugeordnet ist, eine Risikostufe zu bestimmen.

11. Kompatibilitätstesteinrichtung nach Anspruch 10,
wobei als Reaktion auf Bestimmen, dass die bestimmte Risikostufe innerhalb eines zulässigen Risikobereichs liegt, die zusätzliche Funktionalität für den Betrieb mit dem medizinischen Bildgebungssystem zulässig ist; und
wobei die Kompatibilitätsprüf-Engine als Reaktion auf Bestimmen, dass das bestimmte Risikoniveau einen zulässigen Risikobereich überschreitet, dazu konfiguriert ist, eine Fehlermeldung zu erzeugen und/oder eine Aktion zum Reduzieren der Risikostufe auszulösen.

12. System (100) zur Kompatibilitätsprüfung einer an ein medizinisches Bildgebungssystem angeschlossenen Komponente, das Folgendes umfasst:
eine Kompatibilitätstesteinrichtung nach einem der vorstehenden Ansprüche; und
- ein Bildgebungs-Phantom mit einer Sensoranordnung;
wobei die Sensoranordnung einen oder mehrere Sensoren umfasst, die dazu konfiguriert sind, Sensordaten zu erheben, die auf eine Leistung des medizinischen Bildgebungssystems hinweisen.

13. Medizinisches Bildgebungssystem (30), das Folgendes umfasst:
- eine Kompatibilitätstesteinrichtung nach einem der Ansprüche 1 bis 11 oder ein System nach Anspruch 12.

14. Verfahren (200) zum Betreiben einer Kompatibilitätstesteinrichtung nach Anspruch 1 zur Kompatibilitätsprüfung einer an ein medizinisches Bildgebungssystem angeschlossenen Komponente, wobei das Verfahren umfasst:
- Anschließen eines Konnektivitätsmoduls der Kompatibilitätstesteinrichtung an eine Kompatibilitätstestschnittstelle des medizinischen Bildgebungssystems; und
- Durchführen eines oder mehrerer Kompatibilitätstests, um zu bestimmen, ob die angeschlossene Komponente mit dem medizinischen Bildgebungssystem kompatibel ist.

## Revendications

1. Appareil de test de compatibilité (10) pour la vérification de compatibilité d'un composant (20) pouvant être connecté à un système d'imagerie médicale (30), l'appareil de test de compatibilité comprenant :
- un module de connectivité (12) ; et
- un moteur de vérification de compatibilité (14) ;
dans lequel le module de connectivité peut être connecté au système d'imagerie médicale ;
dans lequel le moteur de vérification de compatibilité est configuré pour effectuer un ou plusieurs tests de compatibilité afin de déterminer une compatibilité entre le composant connecté et le système d'imagerie médicale et pour générer un résultat associé aux un ou plusieurs tests de compatibilité ;
dans lequel le système d'imagerie médicale est configuré pour échanger des données avec le composant connecté par l'intermédiaire d'une interface de données (42) conformément à un protocole de communication ; et
dans lequel les un ou plusieurs tests de compatibilité comprennent un test de compatibilité du composant connecté avec l'interface de données et/ou le protocole de communication.

2. Appareil de test de compatibilité selon la revendication 1,
dans lequel le moteur de vérification de compatibilité est configuré pour effectuer une ou plusieurs des étapes suivantes :
- la surveillance d'une communication de données entre le système d'imagerie médicale et le composant connecté, et la détermination si la communication de données suit un protocole de communication prédéterminé ;
- la communication avec le composant connecté à l'aide d'un message pour obtenir une réponse, et la comparaison de la réponse obtenue à une réponse souhaitée afin de déterminer si le composant connecté est compatible avec le système d'imagerie médicale ; et
- le déclenchement d'un événement représentant un changement ou une activité qui a lieu dans un environnement de fonctionnement du système d'imagerie médicale, la surveillance d'une réaction du composant connecté à l'événement et la détermination si le composant connecté est compatible avec le système d'imagerie médicale sur la base d'un modèle action-réaction.

3. Appareil de test de compatibilité selon l'une quelconque des revendications précédentes,
dans lequel les un ou plusieurs tests de compatibilité comprennent une vérification de fonctionnalité pour déterminer si le composant connecté prend en charge une fonctionnalité supplémentaire.

4. Appareil de test de compatibilité selon la revendication 3,
dans lequel, en réponse à la détermination que le composant connecté ne prend pas en charge la fonctionnalité supplémentaire, l'appareil de test de compatibilité est configuré pour effectuer une ou plusieurs des étapes suivantes :
- la désactivation de la fonctionnalité supplémentaire pour le composant connecté ;
- le blocage d'une communication entre le composant connecté et le système d'imagerie médicale ; et
- la configuration du système d'imagerie médicale selon un réglage différent pour prendre en charge la fonctionnalité supplémentaire du composant connecté.

5. Appareil de test de compatibilité selon la revendication 3 ou 4,
dans lequel, en réponse à la détermination que le composant connecté prend en charge la fonctionnalité supplémentaire, l'appareil de test de compatibilité est configuré pour effectuer une ou plusieurs des étapes suivantes :
- l'activation de la fonctionnalité supplémentaire pour le composant connecté ;
- la réalisation d'une traduction de message validée pour activer la fonctionnalité supplémentaire dans le composant connecté, lorsqu'il est déterminé que le composant connecté présente une fonctionnalité similaire à la fonctionnalité supplémentaire, mais avec un protocole de communication différent ; et
- la fourniture des informations sur le composant connecté et un réglage du système d'imagerie médicale.

6. Appareil de test de compatibilité selon l'une quelconque des revendications précédentes,
dans lequel les un ou plusieurs tests de compatibilité comprennent une mesure d'une caractéristique de performance du système d'imagerie médicale avec le composant connecté pour déterminer si la caractéristique de performance mesurée se trouve à un niveau de qualité défini.

7. Appareil de test de compatibilité selon la revendication 6,
dans lequel le moteur de vérification de compatibilité est configuré pour effectuer une ou plusieurs des étapes suivantes :
- la réception des données de capteur obtenues à partir d'un fantôme d'imagerie, la détermination d'une performance du système d'imagerie médicale sur la base des données du capteur et la détermination si la performance du système d'imagerie médicale répond au niveau de qualité défini ; et
- la réception des données d'image d'un fantôme d'imagerie obtenues par le système d'imagerie médicale, la détermination d'une qualité d'image des données d'image obtenues et la détermination si la qualité d'image répond au niveau de qualité défini.

8. Appareil de test de compatibilité selon l'une quelconque des revendications précédentes,
dans lequel les un ou plusieurs tests de compatibilité comprennent une simulation de données hors de la plage pour vérifier la réaction du composant connecté.

9. Appareil de test de compatibilité selon l'une quelconque des revendications précédentes,
dans lequel les un ou plusieurs tests de compatibilité comprennent un test de compatibilité d'un composant tiers connu et certifié pour générer un résultat du test de compatibilité.

10. Appareil de test de compatibilité selon l'une quelconque des revendications précédentes,
dans lequel le moteur de vérification de compatibilité est configuré pour déterminer un niveau de risque sur la base du résultat associé aux un ou plusieurs tests de compatibilité.

11. Appareil de test de compatibilité selon la revendication 10,
dans lequel, en réponse à la détermination que le niveau de risque déterminé se trouve dans la plage de risque admissible, la fonctionnalité supplémentaire peut être autorisée à fonctionner avec le système d'imagerie médicale ; et
dans lequel, en réponse à la détermination que le niveau de risque déterminé dépasse une plage de risque admissible, le moteur de vérification de compatibilité est configuré pour générer un message d'erreur et/ou déclencher une action pour réduire le niveau de risque.

12. Système (100) de vérification de compatibilité d'un composant connecté à un système d'imagerie médicale, comprenant :
- un appareil de test de compatibilité selon l'une quelconque des revendications précédentes ; et
- un fantôme d'imagerie avec un agencement de capteurs ;
dans lequel l'agencement de capteurs comprend un ou plusieurs capteurs configurés pour obtenir des données de capteur indiquant une performance du système d'imagerie médicale.

13. Système d'imagerie médicale (30), comprenant :
- un appareil de test de compatibilité selon l'une quelconque des revendications 1 à 11 ou un système selon la revendication 12.

14. Procédé (200) de fonctionnement d'un appareil de test de compatibilité selon la revendication 1 pour la vérification de compatibilité d'un composant connecté à un système d'imagerie médicale, le procédé comprenant :
- la connexion d'un module de connectivité de l'appareil de test de compatibilité à une interface de test de compatibilité du système d'imagerie médicale ; et
- la réalisation d'un ou plusieurs tests de compatibilité pour déterminer si le composant connecté est compatible avec le système d'imagerie médicale.
